# EUROPÄISCHE PATENTANMELDUNG

(11) **EP 0 728 723 A2**
(43) Veröffentlichungstag der Anmeldung: **28.08.1996**
(21) Anmeldenummer: 95119623.7
(22) Anmeldetag: 13.12.1995
(51) Int. Cl.: C07C 37/20, C07C 39/17

(54) **Verfahren zur Herstellung von Fluorenbisphenol**

(30) Priorität: 22.02.1995 DE 19506055
(71) Anmelder: Weyl GmbH, D-68305 Mannheim (DE)
(72) Erfinder: Biedenbach, Bruno, Dr., D-67547 Worms (DE); Orth, Winfried, Dr., D-67454 Hassloch (DE); Weiss, Wolfgang, Dr., D-68535 Edingen-Neckarhausen (DE)
(74) Vertreter: Patentanwälte Zellentin & Partner

(57) **Zusammenfassung**

Die Erfindung betrifft ein Verfahren zur Herstellung von 9,9-Bis-(4-hydroxyphenyl-)fluoren durch eine durch β-Mercaptopropion- oder Mercaptoessigsäure katalysierte Kondensation von Fluorenon mit Phenol in stark saurem Medium. Dabei erfolgt die Umsetzung in zwei Stufen:
a. eine Kondensationsreaktion im Temperaturbereich von 55-65° C,
b. eine Isomerisierungsreaktion im Temperaturbereich von 75-100° C.

## Beschreibung

Die Erfindung betrifft ein verbessertes Verfahren zur Herstellung von 9,9-Bis-(4-hydroxyphenyl-)fluoren, in der chem. Technik auch als Fluorenbisphenol bezeichnet, durch Kondensation von Phenol und Fluorenon in stark saurem Medium.

Derartige Verfahren sind seit 1970 (US-A 3,546,165) bekannt und vielfach variiert worden, wobei die Variationen vornehmlich in der Abtrennung und Reinigung des Reaktionsproduktes und in der Auswahl der katalytischen Bedingungen zur Kondensationsreaktion liegen.

Bei den wohl elegantesten Verfahren, die in EP-A 0 502 252 und EP-A 0 502 253 beschrieben sind, wird das stark saure Medium durch Einleiten von HCl-Gas erzeugt und die Abtrennung des Reaktionsproduktes erfolgt durch Adduktbildung mit einem Polyalkylenglykol oder einem Nitril.

Allen bekannten Verfahren, auch den zuletzt genannten, haftet der Nachteil an, daß sich bei der Kondensationsreaktion bis zu 20 %, bezogen auf das eingesetzte Fluorenon, Nebenprodukte, vorwiegend o,p-Isomere und Trimere bilden, die nach der Aufarbeitung des Reaktionsgemisches als Rückstand verbleiben und entsorgt werden müssen.

Es ist daher Aufgabe der Erfindung, ein Verfahren bereitzustellen, bei dem der Anfall an unerwünschten Nebenprodukten minimiert ist.

Die Lösung der Aufgabe erfolgt durch ein Verfahren gemäß der Ansprüche 1-6.

Die Kondensationsreaktion läßt sich im Temperaturbereich von 30-100° C durchführen, wobei, der allgemeinen Regel folgend, die Reaktionsgeschwindigkeit mit steigender Temperatur zunimmt. Mit zunehmender Reaktionstemperatur wird allerdings auch die Bildung der unerwünschten Nebenprodukte sehr stark erhöht. Als optimaler Bereich für eine Reaktionsführung mit wirtschaftlicher Umsetzungsgeschwindigkeit und minimaler Bildung von Nebenprodukten wurde der Temperaturbereich von 55-65° C ermittelt.

Es wurde gefunden, daß nach erfolgter Kondensationsreaktion eine Isomerisierungsreaktion stattfindet, wenn die Temperatur des Reaktionsgemisches auf 75-100° C erhöht wird. Überraschenderweise erfolgt dabei eine bevorzugte Isomerisierung der Nebenkomponenten in Richtung des gewünschten 9,9-Bis-(4-hydroxyphenyl-) fluorens.

Diese Erkenntnis läßt sich auf alle bekannten Verfahren zur Herstellung von Fluorenbisphenol übertragen, indem diese an sich bekannten Umsetzungen in zwei Stufen erfolgen:
a) einer Kondensationsreaktion im Temperaturbereich von 55-65° C, bevorzugt bei 60° C und
b) einer nachfolgenden Isomerisierungsreaktion im Temperaturbereich von 75-100° C.

Die nachfolgende Aufarbeitung des Reaktionsgemisches erfolgt anhand der an sich bekannten Verfahren. Die bevorzugte Aufarbeitung erfolgt durch Adduktbildung mit einem Nitril oder einem Polyalkylenglykol. Der nach der Aufarbeitung des Reaktionsgemisches verbleibende Rückstand kann in einem neuen Reaktionsansatz als Teil der Rohstoffe wieder eingesetzt werden. Dadurch wird die Ausbeute des folgenden Reaktionsansatzes erhöht. Leider läßt sich diese nicht beliebig wiederholen, denn nach etwa dem dritten Reaktionsansatz, bei dem der Rückstand des jeweils vorhergehenden Ansatzes eingesetzt wurde, haben sich nicht isomerisierte Nebenprodukte so weit angereichert, daß dieser Rückstand ausgeschleust und entsorgt werden muß.

Überraschenderweise aber läßt sich auch dieser Verlust an wertvollen Rohstoffen vermeiden, wenn der nach der jeweiligen Aufarbeitung des Reaktionsgemisches verbleibende Rückstand mit Katalysator und Säure versetzt, einer weiteren Isomerisierungsreaktion bei 75-100° C unterworfen wird und danach, ggf. nach Abtrennung der Säure, in einen neuen Reaktionsansatz als Teil der Rohstoffe wieder eingesetzt wird. Eine derartige cyclische Fahrweise bedingt eine weitere Ausbeuteerhöhung und kann mehr als zehnmal wiederholt werden. Auch diese Verfahrensvariante ist im Prinzip auf alle bekannten Verfahren zur Herstellung von Fluorenbisphenol übertragbar. Sie wird jedoch bevorzugt eingesetzt bei den aus EP-A 0 502 252 und EP-A 0 502 253 bekannten Verfahren, bei denen Fluorenon und Phenol in molaren Verhältnissen von 1:4 bis 1:8 vorgelegt werden, mit Mercaptopropionsäure oder -essigsäure katalysiert und durch Einleiten von HCl-Gas in ein stark saures Reaktionsmedium übergeführt werden. Gegenüber diesen Verfahren wurde eine weitere wesentliche Verbesserung gefunden: Während man bisher der Meinung war, die Säure oder saure Komponente zur Einstellung des stark sauren Reaktionsmilieus müsse eine wasserfreie und wasserbindende Substanz sein, um das bei der Kondensationsreaktion entstehende Reaktionswasser zu binden, wurde gefunden, daß auch konzentrierte Salzsäure eingesetzt werden kann. Dies hat gegenüber den aus den o.g. Verfahren bekannten Einleiten von HCl-Gas den Vorteil einer wesentlichen verfahrenstechnischen Verbesserung. Außerdem aber wird dadurch eine deutliche Verkürzung der Reaktionszeit auf 2-3 h (bei 60° C) erzielt.

### BEISPIELE

### Beispiel 1

90 g (0,5 Mol) Fluorenon (99%ig) werden mit 285 g (3 Mol) Phenol vorgelegt und unter Bildung einer klaren Schmelze auf 60° C erhitzt. 1 g 3-Mercaptopropionsäure werden unter Rühren zur Schmelze hinzugefügt und anschließend werden 23 ml Salzsäure (37%ig) innerhalb von 2 h zugetropft. (Exotherme Reaktion). 1 h lang wird bei der Temperatur von 60° C nachreagieren gelassen und danach das Reaktionsgemisch unter Rühren auf 80° C erhitzt. Danach werden 52 g Flüssigkeit, bestehend aus Reaktionswasser, Salzsäure und einem Anteil Phenol, azeotrop bei 80-110° C Badtemperatur und 40-80° C Destillationstemperatur bei einem Druck von 14-18 mm/Hg abdestilliert. Das Reaktionsgemisch wird auf 70° C abgekühlt und man gibt Acetonitril zu, in dem sich der Destillationsrückstand löst. Beim Abkühlen fällt das Addukt aus Fluorenbisphenol und Acetonitril aus, und die Suspension wird unter Rühren auf 20° C gekühlt. Das ausgefallene Addukt wird abfiltriert und bei Raumtemperatur mit Acetonitril portionsweise (3-4mal) gewaschen. Das acetonnitrilfeuchte Addukt wird bei 130° C im Vakuum gespalten und das freie Fluorenbisphenol getrocknet: Ausbeute 124 g = 70,9 %, bezogen auf das eingesetzte Fluorenon; Reinheit: 98,4 %. Aus der Mutterlauge wird das Acetonnitril unter Normaldruck vollständig abdestilliert, und der Destillationsrückstand von - bestehend aus Phenol, Fluorenbisphenol und Nebenkomponenten - der Isomerisierung I zugeführt.

### Beispiel 2

218 g Destillationsrückstand aus Beispiel 1 werden aufgeschmolzen und auf eine Temperatur von 100° C erhitzt. 0,3 ml 3-Mercaptopropionsäure werden unter Rühren zur Schmelze hinzugefügt und anschließend werden 8 ml Salzsäure (37%ig) innerhalb von 0,5 h zugetropft. Das Reaktionsgemisch läßt man bei einer Temperatur von 100° C 7,5 h lang nachreagieren. Danach werden 28 ml Flüssigkeit, bestehend aus Reaktionswasser, Salzsäure und einem Anteil Phenol azeotrop bei 80-110° C Badtemperatur und 40-80° C Destillationstemperatur bei einem Druck von 14-18 mm/Hg abdestilliert. Es verbleiben 195 g Isomerisat I im Destillationsrückstand, die beim nächsten Reaktionsansatz zur Herstellung von Fluorenbisphenol eingesetzt werden.

### Isomerisierungsverlauf:

| Reaktionszeit (h) | Fluorenbisphenol | o,p-Isomer (%) | Trimer (%) |
|---|---|---|---|
| 0 | 55,1 | 21,0 | 22,3 |
| 2 | 76,9 | 15,5 | 6,3 |
| 4 | 82,8 | 12,6 | 3,3 |
| 6 | 84,7 | 10,7 | 4,0 |
| 8 | 85,7 | 9,5 | 3,3 |

### Beispiel 3

Analog zu Beispiel 1 werden 90 g Fluorenon, 188 g Phenol und 195 g Isomerisat I aus Beispiel 2 umgesetzt. Ausbeute: 156 g = 89,2 %; Reinheit: 98,9 %. Als Destillationsrückstand (Rückstand II) werden 235 g einer Mischung erhalten, bestehend aus:
161 g (1,7 Mol) Phenol
74 g Produktgemisch:
53,9 % Fluorenbisphenol
19,8 % o,p-Isomer
24,4 % Trimer.

### Beispiel 4

Analog zu Beispiel 2 werden 235 g Destillationsrückstand aus Beispiel 3 (Rückstand II) isomerisiert. Es verbleiben 223 g Isomerisat II.

### Beispiel 5

Analog zu Beispiel 1 werden 90 g Fluorenon, 142 g Phenol und 223 g Isomerisat II aus Beispiel 4 umgesetzt. Ausbeute an Fluorenbisphenol: 168 g = 96,0 %; Reinheit: 98,7 %. Als Destillationsrückstand (Rückstand III) werden 241 g einer Mischung erhalten, bestehend aus:
160 g (1,7 Mol) Phenol
81 g Produktgemisch:
45,4 % Fluorenbisphenol
20,1 % o,p-Isomer
29,8 % Trimer.

### Beispiel 6

Analog zu Beispiel 2 werden 241 g Destillationsrückstand aus Beispiel 5 (Rückstand III) isomerisiert. Es verbleiben 184 g Isomerisat III.

### Beispiel 7

Analog zu Beispiel 1 werden 90 g Fluorenon, 142 g Phenol und 184 g Isomerisat III aus Beispiel 6 umgesetzt. Ausbeute: 178 g Fluorenbisphenol = 101,7 % bez. auf eingesetztes Fluorenon; Reinheit: 98,2 %. Als Destillationsrückstand (Rückstand IV) werden 185 g einer Mischung erhalten, bestehend aus:
107 g (1,1 Mol) Phenol
78 g Produktgemisch:
42,4 % Fluorenbisphenol
23,2 % o,p-Isomer
37,3 % Trimer.

## Patentansprüche

1. Verfahren zur Herstellung von 9,9-Bis-(4-hydroxyphenyl-)fluoren durch eine durch β-Mercaptopropion- oder Mercaptoessigsäure katalysierte Kondensation von Fluorenon mit Phenol in stark saurem Medium, **dadurch gekennzeichnet**, daß die Umsetzung in zwei Stufen erfolgt:
a. eine Kondensationsreaktion im Temperaturbereich von 55-65° C,
b. eine Isomerisierungsreaktion im Temperaturbereich von 75-100° C.

2. Verfahren nach Anspruch 1, **dadurch gekennzeichnet**, daß der nach der Aufarbeitung des Reaktionsgemisches verbleibende Rückstand in einem neuen Reaktionsansatz als Teil der Rohstoffe wieder eingesetzt wird.

3. Verfahren nach Anspruch 1, **dadurch gekennzeichnet**, daß der nach der Aufarbeitung des Reaktionsgemisches verbleibende Rückstand mit Katalysator und Säure versetzt, einer weiteren Isomerisierungsreaktion bei 75-100° C unterworfen wird und danach ggf. nach Abtrennung der Säure, in einem neuen Reaktionsansatz als Teil der Rohstoffe wieder eingesetzt wird.

4. Verfahren nach einem oder mehreren der Ansprüche 1-3, **dadurch gekennzeichnet**, daß zur Einstellung des stark sauren Mediums konzentrierte Salzsäure verwendet wird.

5. Verfahren nach einem oder mehreren der Ansprüche 1 bis 4, **dadurch gekennzeichnet**, daß die Abtrennung des Reaktionsproduktes aus dem Reaktionsgemisch durch Adduktbildung mit einem Nitril erfolgt.

6. Verfahren nach Anspruch 5, **dadurch gekennzeichnet**, daß die Spaltung des Adduktes aus 9,9-Bis-(4-hydroxyphenyl-)fluoren und Nitril thermisch erfolgt.
